# EUROPEAN PATENT APPLICATION

(11) **EP 4 686 755 A1**
(43) Date of publication of application: **04.02.2026**
(21) Application number: 24461604.1
(22) Date of filing: 29.07.2024
(51) Int. Cl.: C12N 9/10

(54) **PROTEINACEOUS NANOCAPSULES WITH OXIDE-DEPENDENT OPEN-CLOSE CAPABILITY**

(71) Applicant: UNIWERSYTET JAGIELLONSKI, 31-007 Kraków (PL)
(72) Inventor: Azuma, Yusuke, 30-348 Kraków (PL); Malolan Venkatesan, Vishal, 30-348 Kraków (PL); Koziej, Lukasz, 35-705 Kraków (PL)
(74) Representative: Witek, Rafal

(57) **Abstract**

The present invention provides protein cages, formed by novel variants of lumazine synthase from *Aquifex aeolicus* (AaLS), that reversibly change the open-close conformation in response to oxides. This controlled open-close characteristic of the protein cages of the invention provides methods for packaging and release of guest cargo objects under mild physiological conditions.

## Description

### Field of the invention

The present invention relates to protein engineering. In particular, the invention relates to protein cages, formed by novel, engineered variants of lumazine synthase from *Aquifex aeolicus* (AaLS), that reversibly change the conformation in response to oxides, providing methods for packaging and release of guest cargo objects under mild physiological conditions.

### Background of the invention

Hollow proteinaceous particles, referred to as protein cages, are useful for novel biotechnology development.¹⁻³ Prospective applications using such nanocapsules include drug delivery, vaccination, catalysis, and *in-cellulo* production of otherwise unstable biomolecules. To achieve these goals, naturally occurring protein cages, e.g. virus-like particles, have been extensively engineered to endow them with customized functionality.

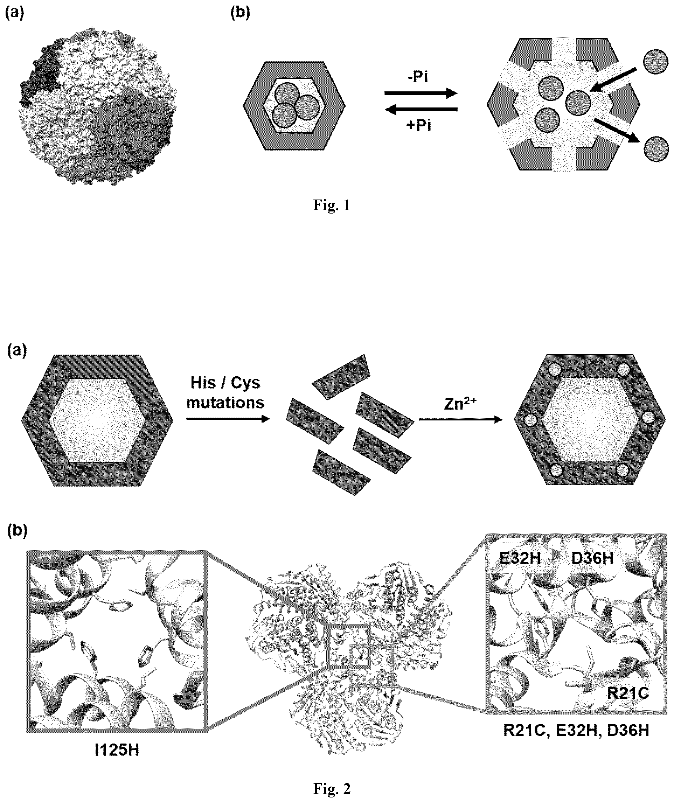

Lumazine synthase (LS), 5-amino-6-(_{D}-ribitylamino)uracil butanedione-transferase (EC 2.5.1.78), derived from several organisms such as *Aquifex aeolicus* is known to form dodecahedral assemblies composed of 60 identical monomers **(****Fig. 1a****)**^{4, 5} Due to extreme thermal stability, facile recombinant production, and high tolerance against both chemical and genetic modification, the enzyme derived from *Aquifex aeolicus* (AaLS) has been extensively employed for diverse applications including biomineralization,⁶ drug delivery/vaccine,⁷⁻⁹ bioimaging,¹⁰ enzyme catalysis,¹¹⁻¹⁴ and as a robust guest encapsulation system.¹⁵ However, AaLS cages are very stable and require harsh conditions to induce their disassembly, hindering package and release of cargo molecules at arbitrary timing and location as required.

Zhang, X.; Meining, W.; Fischer, M.; Bacher, A.; Ladenstein, R., X-ray structure analysis and crystallographic refinement of lumazine synthase from the hyperthermophile Aquifex aeolicus at 1.6 A resolution: determinants of thermostability revealed from structural comparisons. J. Mol. Biol. 2001, 306 (5), 1099-114 discloses the structure of wildtype AaLS. This shell-like structure possesses only ~5Å-side-pseudopentagonal pores at the center of the pentameric subunits, thus limiting influx and outflux of objects larger than the pores.

It has been shown that mutagenesis of AaLS changes the quaternary status of the protein. For example, Sasaki, E.; Böhringer, D.; van de Waterbeemd, M.; Leibundgut, M.; Zschoche, R.; Heck, A. J.; Ban, N.; Hilvert, D., Structure and assembly of scalable porous protein cages. Nat. Commun. 2017, 8, 14663 discloses non-native, porous cage-like structures formed by AaLS mutants called AaLS-neg and AaLS-13. Chen, H. N.; Woycechowsky, K. J, Conversion of a dodecahedral protein capsid into pentamers via minimal point mutations, Biochemistry 2012, 51 (23), 4704-4712 discloses the AaLS mutants that form either non-native, larger cage-like structures or stay as disassembled fragments. However, none of the previously engineered AaLS variants has shown cage conformation change in response to an external stimulus.

Azuma, Y.; Zschoche, R.; Hilvert, D., The C-terminal peptide of Aquifex aeolicus riboflavin synthase directs the encapsulation of native and foreign guests by a cage-forming lumazine synthase. J. Biol. Chem. 2017, 292 (25), 10321-10327 discloses the disassembly of AaLS cages. However, said disassembly requires 3-5 M guanidine hydrochloric acid, which most cargo proteins cannot tolerate.

Bacher, A.; Ludwig, H. C.; Schnepple, H.; Ben-Shaul, Y, Heavy riboflavin synthase from Bacillus subtilis. Quaternary structure and reaggregation. J. Mol. Biol. 1986, 187 (1), 75-86 disclosed morphology changes of lumazine synthase from *Bacillus subtilis* and release of the native guest enzyme, riboflavin synthase, in the effect of removal of phosphate and increase in pH. However, said morphology change requires pH ≥8 and concerns only said protein.

Further, Han, X.; Woycechowsky, K. J., Encapsulation and controlled release of protein guests by the Bacillus subtilis lumazine synthase capsid. Biochemistry 2017, 56 (47), 6211-6220 discloses that encapsulation and controlled release of foreign protein guests by the *Bacillus subtilis* lumazine synthase cage can be obtained in said buffer exchange.

Therefore, a challenge that has remained in the AaLS systems is to induce the cage opening and closing with a mild stimulus that is non-invasive to cargo molecules.

### Summary of the invention

The present invention provides new mutants of lumazine synthase from *Aquifex aeolicus* (AaLS). The amino acid sequence of the wildtype AaLS comprises of:

(SEQ ID NO:1)

MEIYEGKLTA EGLRFGIVAS RFNHALVDRL VEGAIHCIVR HGGREEDITL VRVPGSWEIP

VAAGELARKE DIDAVIAIGV LIRGATPHFD YIASEVSKGL ANLSLELRKP ITFGVITADT

IEQALERAGT KHGNKGWEAA LSAIEMANLF KSLR

The AaLS variants of the invention contain mutations that moderately destabilize the cage-like assembly, which enables the oxide-dependent morphology changes. Specifically, the present invention provides for the AaLS variants consisting of: 
(SEQ ID NO:3)

MEIYEGKLTA EGLRFGIVAS X1FNHALVDRL VX2GAIHCIVX5 X6GGREEDITL VRVPGSWEIP

VAAGELARKE DIDAVIAIGV LIRGATPHFD YIASEVSKGL ANLSLELRKP ITFGVITADT

X3EQAX4ERAGT KHGNKGWEAA LSAIEMANLF KSLR
 wherein: X₁ is cysteine or a cysteine ortholog, X₂ is a non-negatively charged amino acid, at least one of X₃ or X₄ is a non-hydrophobic amino acid, X₅ is arginine or any another amino acid and X₆ is histidine or any another amino acid.

Preferably, the AaLS variants consist of: 
(SEQ ID NO:2)

MEIYEGKLTA EGLRFGIVAS X1FNHALVDRL VX2GAIHCIVR HGGREEDITL VRVPGSWEIP

VAAGELARKE DIDAVIAIGV LIRGATPHFD YIASEVSKGL ANLSLELRKP ITFGVITADT

X3EQAX4ERAGT KHGNKGWEAA LSAIEMANLF KSLR
 wherein X₁, X₂, X₃ and X₄ have been defined above.

The AaLS mutant of invention assembles into cage-like structures that change the conformation from closed to open forms and *vice versa* in response to oxides. Preferably, said oxides are phosphates, phosphate analogs, and organophosphonates.

Specifically, the close form refers the wildtype-like, dodecahedral assembly formed by 12 copies of the pentameric subunits of AaLS. This assembly has only ~5Å-side pseudopentagonal at the center of the pentameric subunits and thus limits in- and outflux of objects larger than the pore.

Specifically, the open forms include cage-like structures, in which one or two of the pentamer-pentamer interaction interfaces remain uncontacted to yield large keyhole-shaped pores in the wall, as well as disassembled cage fragments. Unlike the close form, these opened forms allow access of macromolecules to the cage lumen and release cargoes outside the cages.

The present invention provides a method for changing the conformation of the protein cages formed by the AaLS variants of the inventions. Specifically, the closed-to-opened form transformation of the AaLS cages of the inventions occurs upon removal of oxides, and the opened-to-closed-form transformation of the AaLS cages of the inventions occurs by addition of oxides. Removal of oxides means lowering the oxide concentration of the solution.

The AaLS variants of the invention contain an additional mutation either at position 40 or position 41. Protein cages formed by these mutants disassemble into the cage fragments upon removal of oxides.

The present invention includes a proteinaceous cage-like assembly comprising at least one AaLS variant of the invention. Preferably, other components of said cage-like assembly are circularly permuted variants of AaLS (cpAaLS) that possesses the sequence termini facing to the lumen when assembled.

The present invention includes an inclusion complex comprising protein cages of the invention and cargo molecules, wherein said cargo molecules are encapsulated in said protein cages. Preferably, the cargo molecule is a protein. Further preferably, encapsulation of a protein is achieved by genetic fusion of the cargo to the before-mentioned cpAaLS variants and coassembly with the AaLS variants of the invention.

The present invention provides a method for retrieving guest cargo from the protein cage of the invention. Preferably, said guest retrieval occurs via cleavage of the peptide bond connecting cargo protein and the above-mentioned cpAaLS variants by sequence-specific proteases. Preferably, said protease is the protease derived from tobacco etch virus. An efficient proteolysis reaction occurs only when the protein cages of the invention are opened upon removal of oxides from the solution.

The present invention provides a method for packaging of guest cargo objects in the protein cages of the invention outside cellular contexts. Preferably, said guest cargo objects are small molecules, nucleic acids, proteins, metal nanoparticles, synthetic polymers, and micelles. Preferably, methods for such encapsulation are selected from the group of chemical modification, bioconjugation, enzymatic conjugation, and a combination of these methods, along with oxide-dependent conformation change of the AaLS cages of the invention.

### Description of Figures

Fig. 1 presents the phosphate-dependent conformation change of *Aquifex aeolicus* lumazine synthase (AaLS). (a) Crystal structure of the wildtype AaLS assembly formed by 60 identical subunits (PDB: 1HQK). Individual pentameric units are shown in different colors. (b) A Schematic representation of the phosphate-dependent conformation change of engineered AaLS variants and guest packaging/release.
Fig. 2 presents initial strategy of design of metal-dependent AaLS assembly. (a) Conceptual scheme of reengineering AaLS cage for metal-assisted assembly. (b) Design of AaLS mutant containing R21C, E32H, D36H, and I125H mutations, AaLS^{R21C,E32H, D36H, I125H}. The model was generated using UCSF Chimera X based on the crystal structure of AaLS-wt, 1HQK.

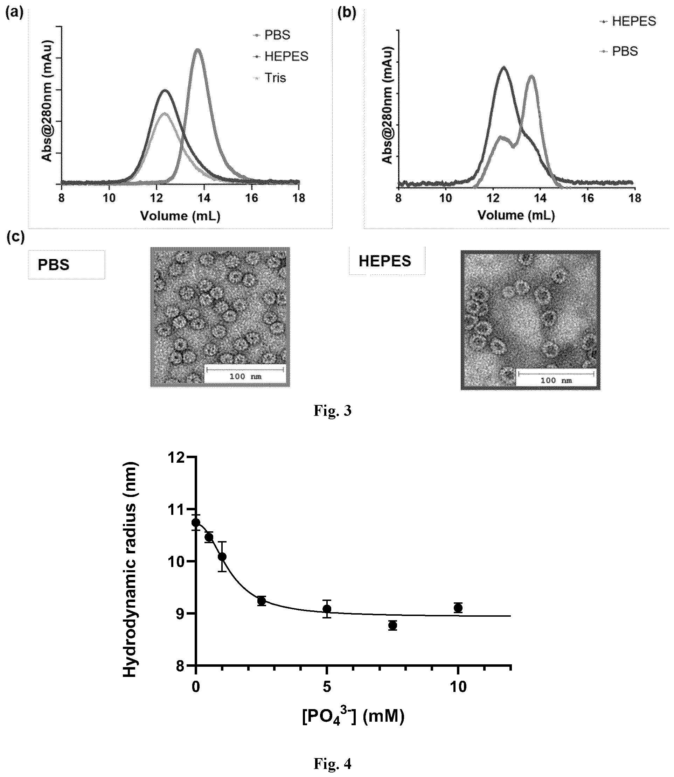

Fig. 3 presents the effective phosphate concentration for the morphology change of AaLS^{R21C,E32H, D36H, I125H}. The half maximal effective concentration of phosphate for the closed to opened form transformation was determined by curve fitting the obtained data points in the Hill's equation, giving 1.3 mM.
Fig. 4 shows morphology change of AaLS^{R21C,E32H, D36H, I125H} by buffer exchange. Size-exclusion chromatogram (a) and negative-stain transmission electron microscope images (c) of the AaLS ^{R21C, E32H, D36H, I125H} variant in the PBS (red), HEPES (blue), or Tris (green) buffer. Restoration of small cage morphology after reintroduction of phosphate in the buffer as shown by size-exclusion chromatography (b).

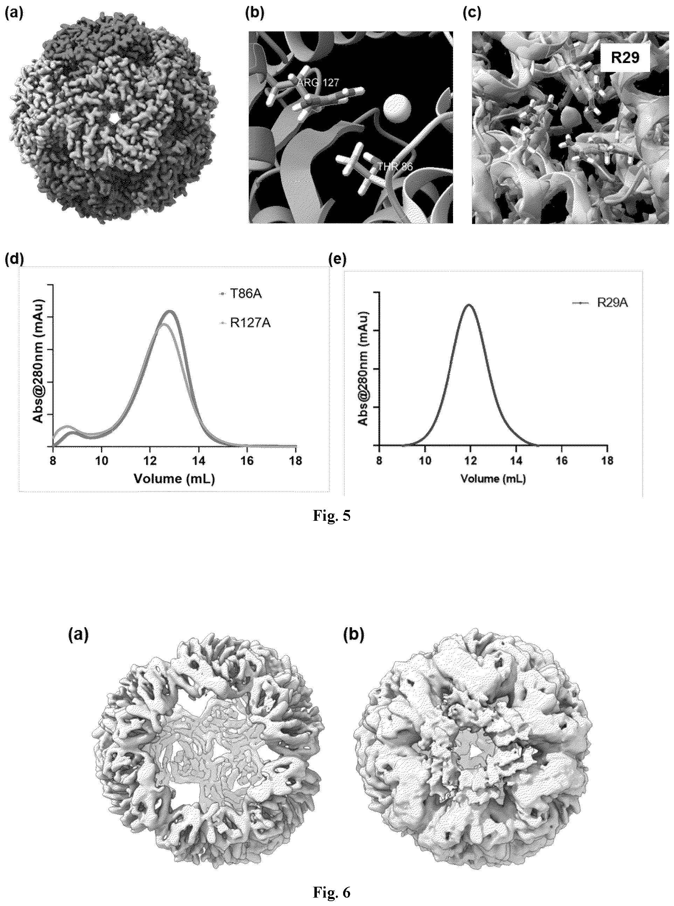

Fig. 5 presents phosphate binding sites of AaLS^{R21C,E32H, D36H, I125H}. (a) CryoEM density map of the entire cage-like structure. (b,c) Amplified image of the native (b) and the newly discovered (c) phosphate binding sites. (d, e) Size-exclusion chromatograph of AaLS^{R21C,E32H, D36H, I125H} containing additional T86A (red) or R127A (green) mutations (d) or R29A mutation (e) in phosphate buffer saline.
Fig. 6 presents preliminary cryoEM structure of AaLS^{R21C,E32H, D36H, I125H} in the HEPES buffer. The same cryoEM map is shown in a high (a) and a low (b) volume threshold.

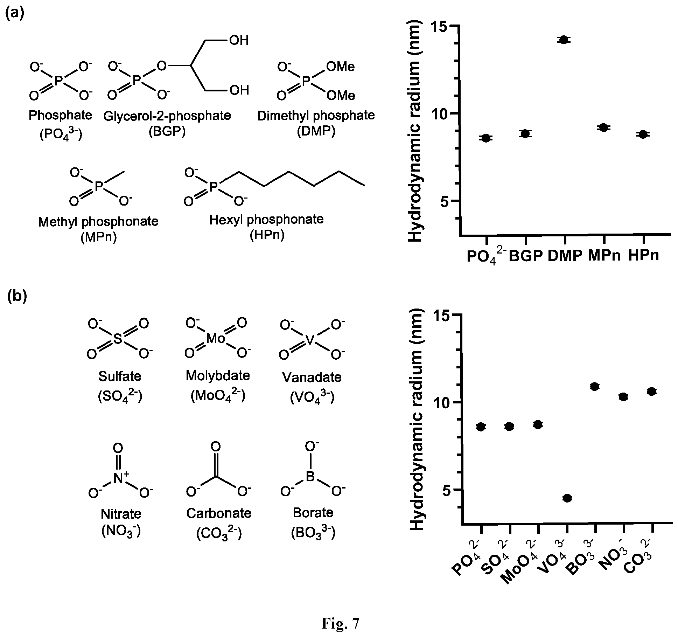

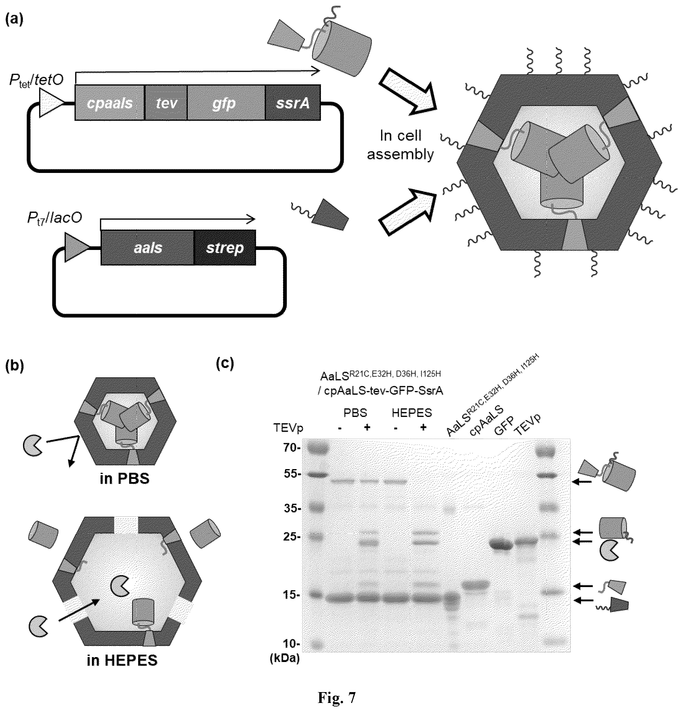

Fig. 7 presents oxide specificity in the cage morphology change, (a) Structure of phosphate, phosphor(mono- or di-)esters, and phosphonates (left) and hydrodynamic radium of AaLS^{R21C,E32H,D36H,I125H} in the presence of these ions (20 mM) (right). (b) Structures of tetro- or trioxides having a similar property to phosphate (left), and hydrodynamic radium of AaLS^{R21C,E32H,D36H,I125H} in the presence of these ions (20 mM) (right). The wildtype-like closed form of the protein cages should exhibit ~9-nm radius, while expanded cages with the open form should be >10 nm. The radius observed with dimethyl phosphate suggested an extralarge cage with a diameter of ~28 nm.

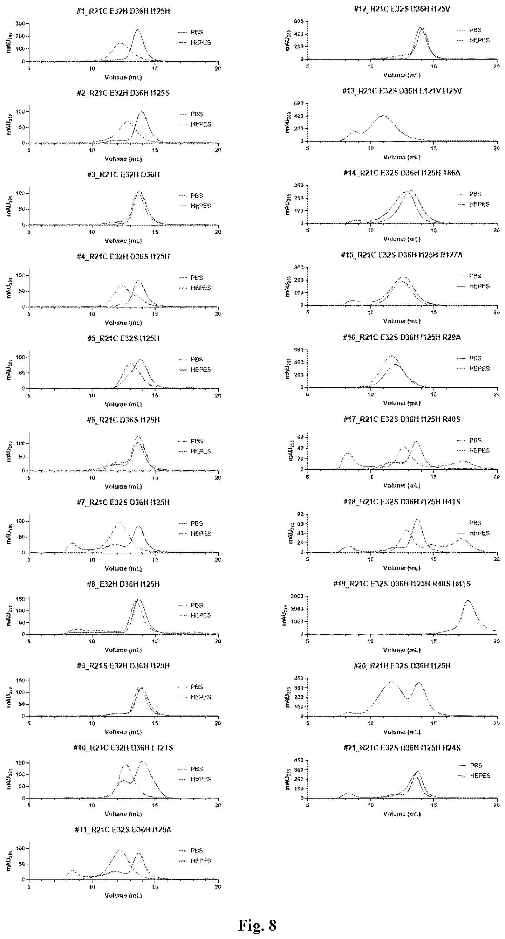

Fig. 8 presents guest protein retrieval using phosphate-dependent morphology change. (a) *In cellulo* guest protein packaging system using circularly permuted variant of AaLS (cpAaLS). The model protein, GFP, was genetically fused to the cpAaLS via the peptide containing TEV protease recognition sequence (tev). The construct was also equipped with a bacterial degradation tag (ssrA) to promote proteolysis unless encapsulation occurs. (b) Schematic representation of how cage morphology change results in proteolytic reaction of external enzymes. (c) SDS-PAGE analysis of the inclusion complex upon treatment with TEV protease (TEVp) in the PBS or the HEPES buffer. Calculated molecular masses of each protein are the following; cpAaLS-tev-GFP-ssrA, 47.6 kDa; GFP-ssrA, 29.0 kDa, TEVp, 26.8 kDa, cpAaLS-tev, 18.5 kDa, and AaLS^{R21C,E32H, D36H, I125H}, 18.1 kDa.
Fig. 9 presents size-exclusion chromatograms of all the tested AaLS mutants in the PBS (Blue) and the HEPES buffer (Red). The PBS, 20 mM sodium phosphate buffer (pH 7.5) containing 150 mM NaCl; the HEPES buffer, 50 mM HEPES-NaOH buffer (pH 7.5) containing 150 mM NaCl. Typical elution volumes for 16-nm (s), 22-24-nm (L), and 28-nm cages (XL) are 13.5-14 mL, 12-13.5 mL, and 10.5-12 mL, respectively. The shoulder accompanying the major 14-mL peak likely corresponds to the dimer or trimer of 16-nm cages.

### Detailed description of the invention

Unless defined otherwise, references to "AaLS", "AaLS protein", and "AaLS variant" include both wildtype and engineered variants of lumazine synthase derived from *Aquifex aeolicus.* The amino acid sequence of the wildtype AaLS comprises of:

(SEQ ID NO:1)

MEIYEGKLTA EGLRFGIVAS RFNHALVDRL VEGAIHCIVR HGGREEDITL VRVPGSWEIP

VAAGELARKE DIDAVIAIGV LIRGATPHFD YIASEVSKGL ANLSLELRKP ITFGVITADT

IEQALERAGT KHGNKGWEAA LSAIEMANLF KSLR

The term protein cages refers to hollow structures formed by multiple copies of proteinaceous building blocks. The term AaLS cages refers to protein cages formed by AaLS proteins. The term AaLS mutants here refers to AaLS variants containing amino acid substitutions from the AaLS-wt sequence.

The AaLS variants of the invention contain mutations that moderately destabilize the assembly, which enables the oxide-dependent morphology changes. Specifically, the present invention provides for the AaLS variants consisting of: 
(SEQ ID NO:2)

MEIYEGKLTA EGLRFGIVAS X1FNHALVDRL VX2GAIHCIVR HGGREEDITL VRVPGSWEIP

VAAGELARKE DIDAVIAIGV LIRGATPHFD YIASEVSKGL ANLSLELRKP ITFGVITADT

X3EQAX4ERAGT KHGNKGWEAA LSAIEMANLF KSLR
 wherein X₁ is cysteine or a cysteine ortholog, X₂ is a non-negatively charged amino acid, and at least one of X₃ or X₄ is a non-hydrophobic amino acid.

The term "cysteine ortholog" refers to amino acids with biochemical properties similar to cysteine. Preferably, said cysteine ortholog interacts with the histidine at position 23. Preferably, said cysteine ortholog is selected from the group of homocysteine, selenocysteine, aspartate, and glutamate. In a further preferred embodiment, X₁ is cysteine.

The term "non-negatively charged amino acid" here refers to amino acids of which the side chains do not have a negatively charged group. Preferably, said non-negatively charged amino acid does not interact with the arginine at position 29. In a further preferred embodiment, X₂ is serine.

The term "non-hydrophobic amino acid" here refers to amino acids of which the side chains do not comprise hydrophobic groups. Examples of hydrophobic amino acids here include leucine, isoleucine, valine, methionine, phenylalanine, and tryptophan. In a preferred embodiment, X₃ or X₄ is selected from the group of glycine, serine, threonine, alanine, cysteine, histidine, aspartate, and glutamate.

The AaLS mutant of invention assembles into cage-like structures that change the conformation from close to open forms and *vice versa* in response to oxides. Specifically, the close-to-open form transformation of the AaLS cages of the invention occurs upon removal of oxides, and the opened-to-closed-form transformation of the AaLS cages of the invention occurs by addition of oxides.

In the context of the present invention, "oxides" refer to binary chemical compounds containing oxygen atoms in their structures. Preferably, said oxide used for open-close transformation of the protein cages of the invention is tetroxide which contains four oxygen atoms in the structures. In a further preferred embodiment, said oxides are phosphates, phosphate analogs, and organophosphonates.

The term "phosphates" here refers to anions, salts, and esters derived from phosphoric acid, described by a chemical formula of H₃PO₄. Said phosphate anions are H₂PO₄, HPO₄²⁻, and PO₄³⁻. Said phosphate salts are phosphoric acid derivatives where one 1, 2, or 3 hydrogens are neutralized by counter bases such as sodium hydroxide, potassium hydroxide, and lithium hydroxide. Said phosphate esters are phosphoric acid derivatives where one hydrogen is substituted by organyl groups. Preferably, said organyl group is an alkyl group. In a further preferred embodiment, said monoalkyl phosphate is glycerophosphate.

The term "phosphate analogs" refers to anions, salts, and esters with chemical properties similar to those of phosphates. Examples of said phosphate analog include molybdate, described by a chemical formula of MO₄²⁻ as an anion form, and sulfate, described by a chemical formula of SO₄²⁻ as an anion form.

The term "organophosphonates" here refers to anions, and salts derived from organophosphorus acid, described by a chemical formula of R-H₂PO₃, where R is any organyl group. Preferably, said organyl group is an alkyl group. In a preferred embodiment, said alkyl phosphonates are methyl phosphonate or hexyl phosphonate. Said phosphonate anions are R-HPO₃⁻ and R-PO₃²⁻. Said phosphate salts are organophosphorus acid derivatives where 1 or 2 hydrogens are neutralized by counter bases such as sodium hydroxide, potassium hydroxide, and lithium hydroxide.

The term "close form" of AaLS cages refers to a protein cage where 12 copies of the pentameric subunits assemble into a dodecahedral arrangement. The largest pore of said closed form of AaLS cage is the approximately 5Å-side pseudopentagonal at the center of the pentameric subunits. Thus, said closed form of AaLS cages limits in- and outflux of objects larger than the pore.

The term "open forms" includes cage-like structures, in which some of the pentamer-pentamer interaction interfaces remain uncontacted to yield large pores in the wall, as well as disassembled cage fragments. Unlike the closed form, these opened forms allow access of macromolecules to the cage lumen.

The present invention provides a method for changing the conformation of the AaLS cages of the invention from the open to the closed form. The AaLS mutants defined in SEQ ID NO:2 assemble into the close form of AaLS cages in the presence of oxides. Preferably, the oxide concentration to retain the close form of the AaLS cage is at least 2 mM. When the concentration of AaLS protein is more than 2 mM, the required oxide concentration to retain the close form of AaLS cage is at least 2 equivalent to the protein concentration.

The present invention provides a method for changing the conformation of the AaLS cages of the invention from the close to the open form. The AaLS mutants defined in SEQ ID NO:2 form protein cages that possess 4-11 nm pores upon removal of oxides. Said removal of oxides includes lowering oxide concentration from the solution. The methods for said oxide removal include ultrafiltration, dialysis, size-exclusion chromatography, and metal chelation. In a preferred embodiment, the closed-to-open form transformation of AaLS cages is induced by lowering oxide concentration to no greater than 10 mM using ultrafiltration.

In an additional embodiment, said removal of oxides includes the addition of an antagonist that binds to the protein of the invention competitively with oxides defined before.

In the context of the present invention, the term "antagonists" refers to chemical compounds that competitively bind to the oxide-binding sites of the protein cage of the invention but do not retain the closed form of the protein cage of the invention. Preferably, said antagonist is phosphodiester or phosphotriester, that are phosphoric acid derivatives where two or three hydrogens are substituted by organyl groups. Preferably, said organyl group is an alkyl group. In another preferred embodiment, said antagonist is vanadate.

In addition to the mutants defined in SEQ ID NO:2, the AaLS variants of the invention contain an additional amino acid substitution either at position 40 or at position 41 to a non-native amino acid as shown in SEQ ID NO3: 
(SEQ ID NO:3)

MEIYEGKLTA EGLRFGIVAS X1FNHALVDRL VX2GAIHCIVX5 X6GGREEDITL VRVPGSWEIP

VAAGELARKE DIDAVIAIGV LIRGATPHFD YIASEVSKGL ANLSLELRKP ITFGVITADT

X3EQAX4ERAGT KHGNKGWEAA LSAIEMANLF KSLR
 wherein X₅ or X₆ is a non-positively charged amino acid or a non-histidine orthologue, respectively.

The term "non-positively charged amino acid" here refers to amino acids of which the side chains do not have a positively charged group. Preferably, said non-positively charged amino acid does not interact with glutamates at positions 5 and 145 stems from another chain of the AaLS variant. In a further preferred embodiment, X₅ is serine.

The term "non-histidine ortholog" refers to amino acids of which the side chain do not have biochemical properties similar to histidine. Preferably, said histidine ortholog does not interact with the amino acid at the same position 41, which stems from another chain of the AaLS variant. In a further preferred embodiment, amino acid at position 41 is serine.

Protein cages formed by the AaLS variants containing an amino acid substitution at position 40 or position 41 assemble into the closed form of AaLS cages in the presence of oxides. Preferably, the oxide concentration to retain the closed form of AaLS cage is at least 2 mM. When the concentration of AaLS protein is more than 2 mM, the required oxide concentration to retain the closed form of AaLS cage is at least 2 equivalent to the protein concentration.

Said closed form of the protein cages formed by the AaLS variants containing an amino acid substitution at position 40 or position 41 is disassembled into the cage fragments upon removal of oxides. The term "cage fragment" here refers to any incomplete protein cages. Preferably, cage fragment of AaLS is pentamer, dimer of pentamers, and trimers of pentamers.

Preferably, the AaLS variants defined by SEQ ID NO 2 and SEQ ID NO 3 have an extension of a short peptide tag, wherein said peptide tag is attached to the C- or N-terminus, preferably the C-terminus of the amino acid sequence. Preferably, said short peptide tag comprises 1- to 40-amino acid residues. In a further preferred embodiment, said short peptide tag is a hexahistidine-tag or a strept-tag consisting of an amino acid sequence of HHHHHH or WSHPQFEK, respectively.

The AaLS variants defined by SEQ ID NO 2 and SEQ ID NO 3 form protein cages either themselves or together with other AaLS variants. AaLS cages formed by two or more AaLS variants are referred to as "patchwork" cages. The other AaLS variants that co-assemble with the AaLS variants of inventions are referred to as "assembly partner". Preferably, said assembly partner for patchwork cage formation is selected from the group of wildtype AaLS-wt (SEQ ID NO 1), AaLS mutants, circularly permuted variants of AaLS (cpAaLS), and mutants of cpAaLS. Preferably, said AaLS mutants are selected from the group of AaLS variants of the inventions (SEQ ID NO2 and NO3), and previously engineered variants including AaLS-neg¹⁶, AaLS-13¹⁷, and AaLS-pos¹⁸. The circularly permuted variants of AaLS refer to AaLS variants with topologically rearranged amino acid sequences¹⁹⁻²¹. Preferably, said cpAaLS variants possesses the sequence termini facing to the lumen when assembled. In a further preferred embodiment, said cpAaLS variants comprise amino acid sequences of:
(SEQ ID NO4) 
MATPHFDYIA SEVSKGLANL SLELRKPITF GVITADTLEQ AIERAGTKHG NKGWEAALSA

IEMANLFKSL RXnMEIYEGKL TAEGLRFGIV ASRFNHALVD RLVEGAIDCI VRHGGREEDI

TLVRVPGSWE IPVAAGELARK EDIDAVIAIG VLIRG
(SEQ ID NO5) 
MTLEQAIERA GTKHGNKGWE AALSAIEMAN LFKSLRXnMEI YEGKLTAEGLR FGIVASRFNH

ALVDRLVEGA IDCIVRHGGR EEDITLVRVP GSWEIPVAAG ELARKEDIDA VIAIGVLIRG

ATPHFDYIAS EVSKGLANLS LELRKPITFG VITAD
wherein Xn is a polypeptide connecting native sequence termini.

Preferably, the number of amino acids composed of the Xn is not shorter than 8. In a further preferred embodiment, Xn is a peptide comprising an amino acid sequence of GTGGSGSS.

Said patchwork cages composed of the AaLS variants of inventions and other AaLS variants provide methods for encapsulation of cargo objects in the lumen of said patchwork cages, as previously demonstrated. The terms of cargo and guest refer in general to any objects of interest to place in lumenal space of protein cages. Preferably, said method for encapsulation of cargo objects in AaLS cages is selected from the group of complimentary charge-driven interaction^{16-18, 20, 22-24}, genetic fusion^{19, 20}, tagging system using the peptide derived from riboflavin synthase²⁵, and combination of these methods. Additionally, methods for encapsulation of cargo objects in AaLS cages are selected from commonly used protein modification methods, including chemical modification and bioconjugation, as well as combinations of these methods.

Oxide-dependent close-to-open transformation of the protein cages of the invention enables release of cargo objects that are packaged in AaLS cages, where the guest packaging occurs in host cells and guest release occurs outside said host cells. The host cells here refer to the cells used for the production of the AaLS protein of the invention. Preferably, the cargo molecule is a protein, and encapsulation of said cargo molecules is achieved by genetic fusion of the cargo to the before-mentioned cpAaLS variants and coassembly with the AaLS variants of the invention in the host cells. Release of cargo protein is achieved by oxide removal to change the AaLS cage formation from closed to open forms, followed by cleavage of the peptide linker connecting cargo protein and cpAaLS variants. Preferably, cleavage of the peptide linker is mediated by sequence-specific proteases. Further preferably, said sequence-specific protease is the protease derived from tobacco etch virus.

Oxide-dependent close-to-open transformation of the protein cages of the invention enables encapsulation of cargo objects outside biological context. Specifically, encapsulation of cargo objects can performed in aqueous solution.

Preferably, the cargo objects are both biological and nonbiological entities. Preferably, said cargo objects are selected from the group of small molecules, proteins, nucleic acids, metal nanoparticles, synthetic polymers, and micelles.

In a preferred embodiment, encapsulation of small molecules is mediated by an enzyme that recognizes a specific peptide and conjugates said peptide with a molecule of interest. Preferably, said enzyme is sortase A and said small molecules contain at least one nucleophilic primary amine, described by a chemical formula of R-NH₂, where R is preferably an organyl group containing either an azide or an alkyne moiety. In another preferred embodiment, encapsulation of proteins, nucleic acids, and metal nanoparticles is assisted by azide-alkyne click chemistry.

### Examples

### Example 1. Obtaining of mutants of lumazine synthase from Aquifex aeolicus, which form protein cages with phosphate-dependent open-close capability

To overcome the above-mentioned problems connected with extremely stable assembly of the AaLS wildtype protein, the inventors initially devised a strategy for controlling the assembly by metal ions **(****Fig. 2a****).** Amino acids that can coordinate divalent metal ions such as Zn(II) were introduced to the AaLS-wt protein at the positions that were supposed to be important for the pentamer-pentamer interaction, yielding an AaLS variant containing the R21C, E32H, D36H, and I125H mutations, referred to as AaLS^{R21C,E32H,D36H,I12SH} **(****Fig. 2b****).** It was expected that this variant could not form the dodecahedral cage-like structure on its own while the addition of metal ions would induce the assembly.

Despite the extensive mutations at the pentamer-pentamer interface, the AaLS^{R21C,E32H,D36H,I125H} was found to assemble into the wildtype-like 16-nm cage assembly without the assistance of metal ions. The variant was equipped with a Strep-tag at the C-terminus, overexpressed in *Escherichia coli* cells, and isolated using Strep-Tactin affinity chromatography. Size-exclusion chromatography analysis of the purified protein exhibited a single peak at ~14 mL, known as that for the wildtype protein assembly **(****Fig. 3a****, red).** The homogeneous 16-nm spherical cage structures were confirmed by negative-stain electron microscopy **(****Fig. 3b****, red).**

The AaLS^{R21C,E32H,D36H,I125H} variant was serendipitously found to change its conformation by buffer exchange from 20 mM sodium-phosphate buffer (pH 7.5) containing 150 mM NaCl, referred to as the PBS herein after, to 50 mM HEPES-NaOH buffer (pH 7.5) containing 150 mM, referred to as the HEPES buffer **(****Fig. 3****).** In the later condition, the protein assembly was eluted at -12.5 mL on size-exclusion chromatography while the TEM imaging shows 22-24 nm heterogeneous hollow assemblies **(****Fig. 3****, blue).** A similar structural change was also observed with 50 mM Tris-HCl buffer (pH 7.5) containing 150 mM NaCl instead of the HEPES buffer **(****Fig. 3a****, green).** These results confirmed that the structural change of the AaLS^{R21C,E32H,D36H,I125H} assemblies is attributed to the presence/absence of phosphate ions.

The phosphate-dependent morphology change of AaLS^{R21C,E32H,D36H,I125H} is reversible. The expanded cage structure in the HEPES buffer was isolated and kept in the PBS. Analysis of the sample by size-exclusion chromatography exhibited reappearance of a peak at ~14 mL **(****Fig. 3b****),** suggesting the reformation of the wildtype-like assembly upon addition of phosphate ions.

To investigate how much phosphate is required for the AaLS^{R21C,E32H,D36H,I125H} to retain the wildtype-like 16-nm spherical cage structure, the protein sample was incubated in a varied ratio of the buffers: 50 mM HEPES-NaOH buffer (pH 7.5) containing 150 mM NaCl and 20 mM sodium phosphate buffer containing 150 mM. Dynamic light scattering showed a hydrodynamic radius of ~9 nm in the presence of 2.5- to 20-phosphate, while an increase in the radius was observed by lowering the phosphate concentration to no greater than 1 mM **(****Fig. 4****).** Half maximal effective concentration of phosphate was determined by a Hill's equation, giving 1.3 mM for the transformation of the protein cages of the invention from the closed to open forms.

It has been known that AaLS proteins assemble into a variety of hollow structures upon reengineering. For example, the lumenal surface of the wild-type protein (*Aa*LS-wt) assembly was endowed with a high negative charge by site-directed mutagenesis and subsequently optimized by directed evolution, yielding variants called *Aa*LS-neg and *Aa*LS-13, respectively.^{16, 17} These engineered variants adopt expanded cage-like assemblies with ~28 nm or ~40 nm diameters, respectively, and keyhole-shaped pores in the wall.²⁶ Their structural characteristics allow efficient loading of positively charged cargo simply by mixing the guests and host cages in aqueous solution.^{12, 22} However, none of these previously engineered AaLS variants are known to form wildtype-like, intact cages under any conditions.

The AaLS^{R21C,E32H,D36H,I125H} variant was originally designed for metal-dependent assembly. Thus, particular histidine/cysteine mutations might not be required, but simple alteration of naturally occurring amino acids at these positions would be sufficient for phosphate-dependent morphology change. Indeed, introduction of serine, instead of histidine, to the position of 32 and 125 of the AaLS^{R21C,E32H,D36H,I125H}, results in the variants showing the phosphate-dependent morphology change as expected **(Table 1, #1, #2, and #7).** For the position 36, even mutagenesis is not required, as changing this residue to either serine or naturally occurring aspartate leads to the variant still showing the phosphate dependency (Table 1, #4, **and** #5). In contrast, mutations at the positions 32 and 125 seem to be essential for the phosphate-dependent morphology change. When they were changed back to the original glutamate or isoleucine, these variants formed wildtype-like cages without phosphate ions **(Table 1, #3 and #6).** Unexpectedly, cysteine at the position 21 appears to be crucial. Reverting the cysteine to an arginine, as found in wild-type cages, and to a serine residue at this position leads to the variant forming wildtype-like cages even without phosphate ions **(Table 1, #8 and #9).** Furthermore, replacement of the cysteine with a histidine resulted in the formation of expanded cages even in the presence of phosphate ions **(Table 1, #20).**

**Table 1. AaLS mutants and their formation in the PBS or the HEPES buffer.**

| Variant | | | | | | | | | | | | Formation | |
|---|---|---|---|---|---|---|---|---|---|---|---|---|---|
| | 21 | 32 | 36 | 125 | 121 | 86 | 127 | 29 | 40 | 41 | 24 | PBS | HEPES |
| wt | R | E | D | I | L | T | R | R | R | H | H | S*1 | S |
| #1 | **C** | **H** | **H** | **H** | L | T | R | R | R | H | H | S | L^{*2} |
| #2 | C | H | H | S | L | T | R | R | R | H | H | S | L |
| #3 | C | H | H | I | L | T | R | R | R | H | H | S | S |
| #4 | C | H | S | H | L | T | R | R | R | H | H | S | L |
| #5 | C | S | D | H | L | T | R | R | R | H | H | S | L |
| #6 | C | E | S | H | L | T | R | R | R | H | H | S | S |
| #7 | C | S | H | H | L | T | R | R | R | H | H | S | L |
| #8 | R | H | H | H | L | T | R | R | R | H | H | S | S |
| #9 | S | H | H | H | L | T | R | R | R | H | H | S | S |
| #9 | S | H | H | H | L | T | R | R | R | H | H | S | S |
| #10 | C | H | H | I | S | T | R | R | R | H | H | S | L |
| #11 | C | H | H | A | L | T | R | R | R | H | H | S | L |
| #12 | C | H | H | V | L | T | R | R | R | H | H | S | S |
| #13 | C | H | H | V | **V** | T | R | R | R | H | H | XL^{*3} | XL |
| #14 | C | H | H | H | L | A | R | R | R | H | H | L | L |
| #15 | C | H | H | H | L | T | A | R | R | H | H | L | L |
| #16 | C | H | H | H | L | T | R | A | R | H | H | XL | XL |
| #17 | C | H | H | H | L | T | R | R | S | H | H | S | L,P^{*4} |
| #18 | C | H | H | H | L | T | R | R | R | S | H | S | L,P |
| #19 | C | H | H | H | L | T | R | R | S | S | H | P | N.D.^{*5} |
| #20 | H | H | H | H | L | T | R | R | R | H | H | S,XL | N.D. |
| #21 | C | H | H | H | L | T | R | R | R | H | S | S | S |

| | | | | | | | | | | | | | |
|---|---|---|---|---|---|---|---|---|---|---|---|---|---|
| *1, Small, wildtype-like 16-nm cages *2, Large, expanded 22-24-nm cages *3, Extra-large, expanded 24-28 nm cages *4, Pentamers *5, Not determined | | | | | | | | | | | | | |

Mutagenesis at the specific positions, 21, 36, and 125, is not only the way to yield phosphate-dependent AaLS cages but modestly destabilizing naturally occurring pentamer-pentamer interaction also results in a similar effect. As such, inventors mutated the histidine at position 125 back to the original isoleucine and the leucine at the position 121, which form hydrophobic core at 3-fold symmetry with the isoleucine 125, to serine, AaLS^{R21C,E32H,E36H,L121S}, which still showed a phosphate-dependent morphology change like the parent variant **(Table 1, #10).**

Importance of the hydrophobic core was further confirmed with alanine or valine mutation at the position 125, AaLS^{R21C,E32H,E36H,I125A}, AaLS^{R21C,E32H,E36H,L125V}. While the former variants with a mutation possessing small side chain showed a phosphate-dependent morphology change, another with valine, the side chain is only one methyl group shorter compared to the parent leucine, forms the wildtype-like assembly regardless of the buffers **(Table 1, #11 and #12).** These results suggested that destabilization of the 3-fold symmetry hydrophobic core is essential for inducing cage expansion by removal of phosphate. However, replacement of both the hydrophobic residues at position 121 and 125 to valine resulted in the formation of phosphate-independent cages with diameter >24-nm **(Table 1, #13).**

To gain structural insight into the phosphate-dependent morphology change, inventors analyzed the AaLS^{R21C,E32H,E36H,I125H} assembly in the presence of phosphate ions using cryoEM single particle reconstruction. Like the wildtype protein, this mutant assembles into dodecahedral cage-like structures composed of 60 monomer subunits as expected **(****Fig. 5a****).** Phosphate is one of the products in the metabolic reaction performed by lumazine synthase and the crystal structure of AaLS-wt contains a phosphate ion at the catalytic site.^{5, 27} Likewise, a substantial density, likely phosphate ion, was observed at the catalytic sites in the cryoEM structure **(****Fig. 5b****).** Additionally, another ion interacting with three arginine residues at the 3-fold symmetry was also found. This is probably phosphate ion as well, considering the possible interaction mode with arginine **(****Fig. 5c****).** Notably, this phosphate-arginine interaction is unique for the AaLS^{R21C,E32H,E36H,I125H}, and not observed for the crystal structure of the wildtype AaLS assembly.

To test how the naturally occurring and newly discovered phosphate binding sites have an impact on the phosphate-dependent morphology change, the amino acid residues of which the side chains interact with phosphate ions was mutated to alanine. The AaLS^{R21C,E32H,E36H,I125H}derivatives containing additional mutations, T86A, R127A,or R29A at the catalytic sites or the 3-fold symmetry axis showed only expanded structures even in the presence of phosphate ions **(****Fig. 5e** **and Table 1, #14 and #15).** These results suggested that all the corresponding residues interacting with phosphate ions are essential for the maintenance of the wildtype-like morphology.

Moreover, the importance of the cysteine residue at position 21 is substantiated with the observation that it has interactions with two of the neighboring histidine residues at positions 24 and 36 **(****Fig 5d****).** While the residue at position 36 was found to be inconsequential to phosphate-dependent morphology change **(Table 1, #4-6),** the consequence of disturbing the interaction of the cysteine with the native histidine was studied by replacing it with serine **(Table 1, #21).** This variant remained as intact wild-type cages even in the absence of phosphate, suggesting that the C21-H24 interaction is paramount for cage expansion upon phosphate removal.

In an effort to completely deconstruct the capsid structures into pentameric building blocks, point mutations described in a previous study on AaLS capsid conversion to pentamers were introduced into the expanding variants.³⁵ Serine mutations to the AaLS^{R21C,E32H,E36H,I125H} variant at either the arginine and histidine side chains at positions 40 and 41, respectively **(Table 1, #17, #18),** demonstrated that upon phosphate removal and subsequent size-exclusion chromatography, there was a significant fraction that completely disassembled into pentameric subunits, observed as a peak at -17.5 ml. However, the variant with the presence of both mutations **(Table 1, #19)** prevents the formation of intact cage structures, even in the presence of phosphate in the solution.

Many of the previously engineered and structurally characterized AaLS variants assemble into porous cage-like structures. They are formed by the pentameric subunits with 1-3 interfaces remain uncontacted with neighboring capsomers. While there is one AaLS variant that adopts a triangular number *T* = 3 assembly containing hexameric subunits,²³ this one has been heavily reengineered for packaging its own mRNA in the lumen, including circular permutation, fused to RNA-binding site at the N-terminus, followed by multiple rounds of directed evolution. Therefore, inventors assumed that the cage-like structures formed by the AaLS^{R21C,E32H,E36H,I125H} variant contain large pores in the structure.

CryoEM single particle reconstruction of the assemblies in the absence of phosphate ions indeed indicated a structure possessing large gaps between the pentameric subunits **(****Fig. 6a****),** and the density in the large pore region can be visible only when the volume threshold is decreased **(****Fig. 6b****).** These results suggested that the protein in the region is highly dynamic and flexible, likely allowing in- and outflux of molecules across the protein cage wall.

### Example 2. Controlling the conformation of the protein cage of the invention using various oxides

The newly introduced phosphate-binding site formed by three arginine residues at position 29 suggested that oxides possessing similar chemical properties to phosphate could be used for morphology change of the protein cages of the invention. To test this possibility, AaLS^{R21C,E32H,D36H,I125H} was incubated in 25 mM HEPES buffer (pH 7.5) containing 150 mM NaCl and 20 mM oxides of interest, and the hydrodynamic radius was measured by DLS **(****Fig. 7a,b).** A hydrodynamic radius of ~9 nm was observed with phosphate and a phosphomonoester, glycerol-2-phosphate, indicating the wildtype-like, closed form of the particles. Meanwhile, a phosphodiester, dimethyl phosphate, changed the radius to ~11 nm, suggesting cage expansion to the open form. Retaining the closed form of the AaLS^{R21C,E32H,D36H,I125H} was also observed with phosphonates, methyl phosphonate and hexyl phosphonate. These results suggest that at least 3 oxygen atoms of phosphate should remain unmodified with organyl groups to retain the wildtype-like 16-nm assembly of AaLS^{R21C,E32H,D36H,I125H}.

Oxides with similar chemical properties to phosphate can retain the closed form of the AaLS^{R21C,E32H,D36H, I125H} assemblies. Specifically, sulfate and molybdate were confirmed to serve as phosphate alternative for the protein. In the presence of these oxides, AaLS^{R21C,E32H,D36H, I125H} shows a hydrodynamic radius of ~9 nm, corresponding to the closed form of the protein cages. In contrast, borate, nitrate, and carbonate could not retain the closed form, and the variant exhibited ~11-nm hydrodynamic radius, indicating cage expansion to the open form. Interestingly, vanadate, which is frequently used as a phosphate analogue, changes the hydrodynamic radius of the protein to ~4 nm, suggesting cage disassembly to pentameric subunits.

### Example 3. Using of protein cages with phosphate-dependent open-close capability

The utility of the intact to porous assembly rearrangement has been demonstrated through the retrieval of packaged proteins using a sequence-specific protease derived from tobacco mosaic virus, TEVp. A green fluorescent protein possessing a bacterial degradation tag, GFP-ssrA, was genetically fused to a circularly permuted AaLS via a TEVp recognition peptide, and coproduced with the AaLS^{R21C,E32H,E36H,I125H} variant in *E. coli* cells **(****Fig. 7a****),** the guest packaging system has been recently described.¹⁹ The patchwork assemblies were isolated with affinity chromatography, followed by treatment with TEVp in the absence of presence of phosphate ions. SDS-PAGE analysis of the protease-treated samples suggested that the efficient cleavage occurred only in the absence of phosphate ions **(****Fig. 7b****),** confirming that the protease can internalize the cage in the absence of phosphate while the intact shell in the presence of phosphate prevents the enzyme entry to the lumen.

### Example 4. Materials and methods

### Materials

Chemicals and biochemicals were purchased from Sigma Aldrich (Merck, Darmstadt, Germany), New England Biolabs (Ipswich, MA, USA), or Thermo Fisher Scientific (Waltham, MA, USA). Oligonucleotides were purchased from Sigma Aldrich. Synthetic plasmid pET21 containing gene encoding AaLS^{R21C,E32H,D36H,I125H}, pET21a_AaLS_R21C_E32H_D36H_I125H-StreptII, was procured from BioCat GmbH (Heidelberg, Germany). *E*. *coli* BL21-Gold(DE3) and DH5α competent cells were purchased from Agilent Technologies (Santa Clara, U.S.A.) and Thermo Fisher Scientific, respectively.

### Molecular cloning

Quick-change PCR for site-directed mutagenesis was performed using pET21a_AaLS_R21C_ E32H_D36H_I125H-StreptII as template and corresponding primer pairs shown in Table 2. PCR was performed using a Phusion polymerase on an Eppendorf Master cycler Nexus thermal cycler. The resulting reaction solution was treated with Dpnl at 37°C for 2 hours, and was directly used for transformation of *E. coli* strain DH5α. Sequences of plasmids were confirmed by DNA Sanger sequencing, performed by Eurofins Genomics GmbH (Ebersberg, Germany).

**Table 2. Plasmids used for example experiments**

| **Name** | **Vector** | **Gene** | **C-tag** | **Marker^{a}** | **Ori** | **Promoter^{b}** | **Operon^{c}** | **Regulation gene^{e}** |
|---|---|---|---|---|---|---|---|---|
| pET21a_AaLS_R21C_E32H_ D36H_I125H-StreptII | pET21a (+) | AaLS_R21C_E32H_D3 6H_I125H | Strept II | Amp^{R} | ColE1 | *P*_{T7} | *LacO* | *Lac*I |
| pET21a_AaLS_R21C_E32H_ D36H-R40S-H41S-I125H-Streptll | pET21a (+) | AaLS_R21C_E32H_D3 6H-R40S-H41S-I125H | Strept II | Amp^{R} | ColE1 | *P*_{T7} | *LacO* | *Lac*I |
| pET21a_AaLS_R21C_E32H_ D36H-StreptII | pET21a (+) | AaLS_R21C_E32H_D3 6H | Strept II | Amp^{R} | ColE1 | *P*_{T7} | *LacO* | *Lac*I |
| pET21a_AaLS_R21C_E32H_ D36H_I125S-StreptII | pET21a (+) | AaLS_R21C_E32H_D3 6H 1125S | Strept II | Amp^{R} | ColE1 | *P*_{T7} | *LacO* | *Lac*I |
| pET21a_AaLS_R21C_E32H_ D36S_I125H-StreptII | pET21a (+) | AaLS_R21C_E32H_D3 6S_I125H | Strept II | Amp^{R} | ColE1 | *P*_{T7} | *LacO* | *Lac*I |
| pET21a_AaLS_R21C D36S_ 1125H-Streptl I | pET21a (+) | AaLS_R21C_D36S_I12 5H | Strept II | Amp^{R} | ColE1 | *P*_{T7} | *LacO* | *Lac*I |
| pET21a_AaLS_R21S_E32H_ D36H_I125H-StreptII | pET21a (+) | AaLS_R21S_E32H_D3 6HJ125H | Strept II | Amp^{R} | ColE1 | *P*_{T7} | *LacO* | *Lac*I |
| pET21a_AaLS_R21C_E32S_ 1125H-Streptl I | pET21a (+) | AaLS_R21C_E32S_I12 5H | Strept II | Amp^{R} | ColE1 | *P*_{T7} | *LacO* | *Lac*I |
| pET21a_AaLS_R21C_E32H_ D36H_L121S-StreptII | pET21a (+) | AaLS_R21C_E32S_L1 21S | Strept II | Amp^{R} | ColE1 | *P*_{T7} | *LacO* | *Lac*I |
| pET21a_AaLS_R21C_E32H_ D36H_I125A-StreptII | pET21a (+) | AaLS_R21C_E32S_I12 5A | Strept II | Amp^{R} | ColE1 | *P*_{T7} | *LacO* | *Lac*I |
| pET21a_AaLS_R21C_E32H_ D36H_I125V-StreptII | pET21a (+) | AaLS_R21C_E32S_I12 5V | Strept II | Amp^{R} | ColE1 | *P*_{T7} | *LacO* | *Lac*I |
| pET21a_AaLS_R21C_E32H_ D36H_T86A_I125H-StreptII | pET21a (+) | R21C_E32H_D36H_T8 6A_I125H | Strept II | Amp^{R} | ColE1 | *P*_{T7} | *LacO* | *Lac*I |
| pET21a_AaLS_R21C_E32H_ D36H_I125H_R127A-Streptll | pET21a (+) | R21C_E32H_D36H_I1 25H_R127A | Strept II | Amp^{R} | ColE1 | *P*_{T7} | *LacO* | *Lac*I |
| pET21a_AaLS_R21C_R29A_ E32H_D36H_I125H-StreptII | pET21a (+) | R21C_R29A_E32H_D3 6H_I125H | Strept II | Amp^{R} | ColE1 | *P*_{T7} | *LacO* | *Lac*I |
| pET21a_AaLS_R21C_E32H_ D36H_L121V_I125V-StreptII | pET21a (+) | R21C_E32H_D36H_L1 21V_I125V | Strept II | Amp^{R} | ColE1 | *P*_{T7} | *LacO* | *Lac*I |
| pET21a_AaLS_R21C_E32H_ D36H_R40S_I125H-Streptll | pET21a (+) | R21C_E32H_D36H_R4 0S_I125H | Strept II | Amp^{R} | ColE1 | *P*_{T7} | *LacO* | *Lac*I |
| pET21a_AaLS_R21C_E32H_ D36H_H41 S_I125H-Streptl I | pET21a (+) | R21C_E32H_D36H_H4 1S_I125H | Strept II | Amp^{R} | ColE1 | *P*_{T7} | *LacO* | *Lac*I |
| pET21a _AaLS_E32H_D36H_ 1125H-Streptl I | pET21a (+) | AaLS_E32H_D36H_I12 5H | Strept II | Amp^{R} | ColE1 | *P*_{T7} | *LacO* | *Lac*I |
| pET21a_AaLS_R21C_E32S_ D36H_I125H-StreptII | pET21a (+) | AaLS_R21C_E32S_D3 6H_I125H | Strept II | Amp^{R} | ColE1 | *P*_{T7} | *LacO* | *Lac*I |
| pET21a_AaLS_R21C_R24S_ E32H_D36H_I125H-StreptII | pET21a (+) | AaLS_R21C_E32S_L1 21S | Strept II | Amp^{R} | ColE1 | *P*_{T7} | *LacO* | *Lac*I |
| pACYC184_cpAaLS(119)_TE Vrs_msfGFP_ssrA | pACYC 184 | cpAaLS(119)-TEVrs-msfGFP-ssrA | - | C M | p15A | *P*ₜₑₜ | *TetO* | *TetR* |

| | | | | | | | | |
|---|---|---|---|---|---|---|---|---|
| ^{a} Amp^{R}, β-lactamase; Cm^{R}, chloramphenicol acetyltransferase. ^{b} *P*_{T7}, T7 promoter; *P*ₜₑₜ, tetracycline promoter ^{c} *Lac*O*,* lactose operator; *Tet*O*,* tetracycline operator ^{d} *Lac*I*, Lac* I impresser; *Tet*R, tetracycline regulator | | | | | | | | |

**Table 3. Oligonucleotides used for the example experiments**

| **Name** | **Sequence** |
|---|---|
| FW_XhoI_StreptII_SpeI | TCGAGGGCGGCTGGAGCCATCCGCAGTTcGAAAAgTAA |
| RV_SpeI_StreptII_XhoI | CTAGTTAcTTTTCgAACTGCGGATGGCTCCAGCCGCCC |
| FW_AaLS_I125 | GCTGACACCTTGGAACAGGCTatcGAGCGCGCCGGCAC |
| RV_AaLS_I125 | GTGCCGGCGCGCTCGATAGCCTGTTCCAAGGTGTCAGC |
| FW_AaLS_R21S | CTTCGTTTCGGTATCGTAGCATCAAGCTTTAATCATGCTCTTGTCGACCGTC |
| RV_AaLS_R21S | GACGGTCGACAAGAGCATGATTAAAGCTTGATGCTACGATACCGAAACGAAG |
| FW_AaLS_D36S | CCGTCTGGTGGAGGGTGCAATTTCTTGCATAGTCCGTCATGGCGG |
| RV_AaLS_D36S | CCGCCATGACGGACTATGCAAGAAATTGCACCCTCCACCAGACGG |
| FW_AaLS_E32HD36S | CCGTCTGGTGCATGGTGCAATTTCTTGCATAGTCCGTCATGGCGG |
| RV_AaLS_E32HD36S | CCGCCATGACGGACTATGCAAGAAATTGCACCATGCACCAGACGG |
| Fw_AaLS_I125S | GCTGACACCTTGGAACAGGCTAgCGAGCGCGCCGGC |
| Rv_AaLS_I125S | GCCGGCGCGCTCGcTAGCCTGTTCCAAGGTGTCAGC |
| Fw_AaLS_E32S | GCTCTTGTCGACCGTCTGGTGAGCGGTGCAATTGATTGCATAGTCCGTC |
| Rv_AaLS_E32S | GACGGACTATGCAATCAATTGCACCGCTCACCAGACGGTCGACAAGAGC |
| Fw_AaLS_E32SD36H | GCTCTTGTCGACCGTCTGGTGTCTGGTGCAATTCACTGCATAGTCCG |
| Rv_AaLS_E32SD36H | CGGACTATGCAGTGAATTGCACCAGACACCAGACGGTCGACAAGAGC |
| Fw_AaLS_L121S | CTTCGGTGTTATTACAGCTGACACCTCTGAACAGGCTATCGAGCGCG |
| Rv_AaLS_L121S | CGCGCTCGATAGCCTGTTCAGAGGTGTCAGCTGTAATAACACCGAAG |
| Fw_AaLS_I125H_R127A | CACCTTGGAACAGGCTCACGAGGCGGCCGGCACAAAACACGGCAAC |
| Rv_AaLS_I125H_R127A | GTTGCCGTGTTTTGTGCCGGCCGCCTCGTGAGCCTGTTCCAAGGTG |
| Fw_AaLS_I125A | GCTGACACCTTGGAACAGGCTGCGGAGCGCGCCGGC |
| Rv_AaLS_I125A | GCCGGCGCGCTCCGCAGCCTGTTCCAAGGTGTCAGC |
| Fw_AaLS_I125V | GCTGACACCTTGGAACAGGCTGTGGAGCGCGCCGGC |
| Rv_AaLS_I125V | GCCGGCGCGCTCCACAGCCTGTTCCAAGGTGTCAGC |
| Fw_AaLS_T86A | CGTTCTCATCAGAGGCGCAGCGCCACATTTCGATTATATCGCCTCTG |
| Rv_AaLS_T86A | CAGAGGCGATATAATCGAAATGTGGCGCTGCGCCTCTGATGAGAACG |
| Fw_AaLS_R21C_R29A | CATGCTTTAATCATGCTCTTGTCGACGCGCTGGTGCATGGTGCAATTCACTGC |
| Rv_AaLS_R21C_R29A | GCAGTGAATTGCACCATGCACCAGCGCGTCGACAAGAGCATGATTAAAGCATG |
| Fw_AaLS_R21 | CTTCGTTTCGGTATCGTAGCATCAcgtTTTAATCATGCTCTTGTCGACCGTC |
| Rv_AaLS_R21 | GACGGTCGACAAGAGCATGATTAAAACGTGATGCTACGATACCGAAACGAAG |
| Fw_AaLS_E32H-D36H-R40S | GCATGGTGCAATTCACTGCATAGTCAGTCATGGCGGCCGTGAAGAAG |
| Rv_AaLS_E32H-D36H-R40S | CTTCTTCACGGCCGCCATGACTGACTATGCAGTGAATTGCACCATGC |
| Fw_D36H-R40S-H41S | CCGTCTGGTGGAGGGTGCAATTCATTGCATAGTCAGTTCTGGCGG |
| Rv-D36H-R40S-H41S | CCGCCAGAACTGACTATGCAATGAATTGCACCCTCCACCAGACGG |
| Fw_L121V-I125V | CTTCGGTGTTATTACAGCTGACACCGTGGAACAGGCTGTGGAGCG |
| Rv_L121V-I125V | CGCTCCACAGCCTGTTCCACGGTGTCAGCTGTAATAACACCGAAG |
| Fw_AaLS_H24S | CGGTATCGTAGCATCATGCTTTAATAGCGCTCTTGTCGACCGTCTGGTG |
| Rv_AaLS_H24S | CACCAGACGGTCGACAAGAGCGCTATTAAAGCATGATGCTACGATACCG |
| Fw-AaLS-R21H | CTTCGTTTCGGTATCGTAGCATCACACTTTAATCATGCTCTTGTCGACCG |
| Rv-AaLS-R21H | CGGTCGACAAGAGCATGATTAAAGTGTGATGCTACGATACCGAAACGAAG |

**Table 4 Amino acid sequence of the variants tested in the example experiments**

| **Variant** | **Amino acid sequence^{a}** |
|---|---|
| AaLS-wt (SEQ ID NO1) | MEIYEGKLTA EGLRFGIVAS RFNHALVDRL VEGAIDCIVR HGGREEDITL VRVPGSWEIP VAAGELARKE DIDAVIAIGV LIRGATPHFD YIASEVSKGL ANLSLELRKP ITFGVITADT LEQAIERAGT KHGNKGWEAA LSAIEMANLF KSLR |
| AaLS_R21C_E32H_ D36H_I125H (SEQ ID NO6) | MEIYEGKLTA EGLRFGIVAS CFNHALVDRL VHGAIHCIVR HGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_R21C_E32H_ D36H_I125S (SEQ ID NO7) | MEIYEGKLTA EGLRFGIVAS CFNHALVDRL VHGAIHCIVR HGGREEDITL VRVPGSWEIP VAAGELARKE DIDAVIAIGV LIRGATPHFD YIASEVSKGL ANLSLELRKP ITFGVITADT LEQASERAGT KHGNKGWEAA LSAIEMANLF KSLR |
| AaLS_R21C_E32H_ D36H | MEIYEGKLTA EGLRFGIVAS CFNHALVDRL VHGAIHCIVR HGGREEDITL VRVPGSWEIP VAAGELARKE DIDAVIAIGV LIRGATPHFD YIASEVSKGL ANLSLELRKP ITFGVITADT LEQAIERAGT KHGNKGWEAA LSAIEMANLF KSLR |
| AaLS_R21C_E32H_ D36S_I125H (SEQ ID NO8) | MEIYEGKLTA EGLRFGIVAS CFNHALVDRL VHGAISCIVR HGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_R21C_E32S_ 1125H (SEQ ID NO9) | MEIYEGKLTA EGLRFGIVAS CFNHALVDRL VSGAIDCIVR HGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_R21C_D36S_ 1125H | MEIYEGKLTA EGLRFGIVAS CFNHALVDRL VEGAISCIVR HGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_R21C_E32S_ D36H_I125H (SEQ ID NO10) | MEIYEGKLTA EGLRFGIVAS CFNHALVDRL VSGAIHCIVR HGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_E32H_D36H_ I125H | MEIYEGKLTA EGLRFGIVAS RFNHALVDRL VHGAIHCIVR HGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_R21S_E32H_ D36H_I125H | MEIYEGKLTA EGLRFGIVAS SFNHALVDRL VHGAIHCIVR HGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_R21C_E32H_ D36H_L121S (SEQ ID NO11) | MEIYEGKLTA EGLRFGIVAS CFNHALVDRL VHGAIHCIVR HGGREEDITL VRVPGSWEIP VAAGELARKE DIDAVIAIGV LIRGATPHFD YIASEVSKGL ANLSLELRKP ITFGVITADT SEQAIERAGT KHGNKGWEAA LSAIEMANLF KSLR |
| AaLS_R21C_E32H_ D36H_I125A (SEQ ID NO12) | MEIYEGKLTA EGLRFGIVAS CFNHALVDRL VHGAIHCIVR HGGREEDITL VRVPGSWEIP VAAGELARKE DIDAVIAIGV LIRGATPHFD YIASEVSKGL ANLSLELRKP ITFGVITADT LEQAAERAGT KHGNKGWEAA LSAIEMANLF KSLR |
| AaLS_R21C_E32H_ D36H_I125V | MEIYEGKLTA EGLRFGIVAS CFNHALVDRL VHGAIHCIVR HGGREEDITL VRVPGSWEIP VAAGELARKE DIDAVIAIGV LIRGATPHFD YIASEVSKGL ANLSLELRKP ITFGVITADT LEQAVERAGT KHGNKGWEAA LSAIEMANLF KSLR |
| AaLS_R21C_E32H_ D36H_L121V_I125V | MEIYEGKLTA EGLRFGIVAS CFNHALVDRL VHGAIHCIVR HGGREEDITL VRVPGSWEIP VAAGELARKE DIDAVIAIGV LIRGATPHFD YIASEVSKGL ANLSLELRKP ITFGVITADT VEQAVERAGT KHGNKGWEAA LSAIEMANLF KSLR |
| AaLS_R21C_E32H_ D36H_T86A_I125H | MEIYEGKLTA EGLRFGIVAS CFNHALVDRL VHGAIHCIVR HGGREEDITL VRVPGSWEIP VAAGELARKE DIDAVIAIGV LIRGAAPHFD YIASEVSKGL ANLSLELRKP ITFGVITADT LEQAHERAGT KHGNKGWEAA LSAIEMANLF KSLR |
| AaLS_R21C_E32H_ D36H_I125H_R127 A | MEIYEGKLTA EGLRFGIVAS CFNHALVDRL VHGAIHCIVR HGGREEDITL VRVPGSWEIP VAAGELARKE DIDAVIAIGV LIRGATPHFD YIASEVSKGL ANLSLELRKP ITFGVITADT LEQAHEAAGT KHGNKGWEAA LSAIEMANLF KSLR |
| AaLS_R21C_R29A_ E32H_D36H_I125H | MEIYEGKLTA EGLRFGIVAS CFNHALVDAL VHGAIHCIVR HGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_R21C_E32H_ D36H_R40S_I125H (SEQ ID NO 13) | MEIYEGKLTA EGLRFGIVAS CFNHALVDRL VHGAIHCIVS HGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_R21C_E32H_ D36H_H41S_I125H (SEQ ID NO 14) | MEIYEGKLTA EGLRFGIVAS CFNHALVDRL VHGAIHCIVR SGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_R21C_E32H_ D36H_R40S_H41S_ 1125H | MEIYEGKLTA EGLRFGIVAS CFNHALVDRL VHGAIHCIVS SGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_R21H_E32H_ D36H_I125H | MEIYEGKLTA EGLRFGIVAS HFNHALVDRL VHGAIHCIVR HGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| AaLS_R21C_H24S_ E32H_D36H_I125H | MEIYEGKLTA EGLRFGIVAS CFNSALVDRL VHGAIHCIVR HGGREEDITL VRVPGSWEIP VAAGELARKE DIDAVIAIGV LIRGATPHFD YIASEVSKGL ANLSLELRKP ITFGVITADT LEQAHERAGT KHGNKGWEAA LSAIEMANLF KSLR |
| cpAaLS(119)-TEVrs-msfGFP-ssrA | MTLEQAIERA GTKHGNKGWE AALSAIEMAN LFKSLRGTGG SGSSMEIYEG KLTAEGLRFG IVASRFNHAL VDRLVEGAID CIVRHGGREE DITLVRVPGS WEIPVAAGEL ARKEDIDAVI AIGVLIRGAT PHFDYIASEV SKGLANLSLE LRKPITFGVI TADAGGAGGS ENLYFQSGGS GSKGEELFTG VVPILVELDG DVNGHKFSVR GEGEGDATNG KLTLKFICTT GKLPVPWPTL VTTLTYGVQC FSRYPDHMKR HDFFKSAMPE GYVQERTISF KDDGTYKTRA EVKFEGDTLV NRIELKGIDF KEDGNILGHK LEYNFNSHNV YITADKQKNG IKANFKIRHN VEDGSVQLAD HYQQNTPIGD GPVLLPDNHY LSTQSKLSKD PNEKRDHMVL LEFVTAAGIT HGMDELYKTN SAANDENYAL AA |

| | |
|---|---|
| ^{a} Proteins used for the example experiments, except for cpAaLS(119)-TEVrs-msfGFP-ssrA, possesses additional sequence of LEGGWSHPQFEK. | |

### Protein production and purification

*E. coli* strain BL21-Gold(DE3), that was transformed with a pET plasmid encoding corresponding AaLS variant, was cultured at 37 °C in 2× yeast extract - tryptone broth (2×YT) medium supplemented with 100 µg/mL ampicillin until the OD₆₀₀ reached ~0.7-0.9, at which point protein production was induced by the addition of isopropyl β-_{D}-1-thiogalactopyranoside (IPTG) (0.2 mM). After culturing at 25 °C for 20 h, cells were harvested by centrifugation at 5,000 ×g, 4 °C for 10 min, and then stored at -20 °C until purification. The cell pellet from 250-mL culture was suspended in 40-mL PBS-E [20 mM sodium phosphate buffer (molar ratio of Na₂HPO₄ to NaH₂PO₄ being 77.4:22.6) containing 150 mM NaCl, 6 mM KCl, and 1 mM ethylenediaminetetraacetate (EDTA)], supplemented with -0.1 mg/mL lysozyme, -0.01 mg/mL DNase I, and 1 mg/mL RNase A. After incubation at 37 °C for 1 h with gentle shaking, the sample was lysed by sonication and cleared by centrifugation at 15,000 ×g, 25 °C. Supernatant was filtered using a 0.45-µm filter and loaded onto 2-mL StrepTactin Sepharose resin in a gravity column. After complete flowthrough, the column was washed with 16 mL PBS-E. The protein was then eluted with 12 mL PBS-E containing 2.5 mM desthiobiotin. Protein concentration was determined using absorbance at 280 nm using the extinction coefficient of AaLS proteins, *ε*₂₈₀ = 19,630 M⁻¹ cm⁻¹.

The AaLS cages encapsulating GFP-SsrA was produced and isolated using an analogous protocol to that for empty cages. In this patchwork assembly, *E*. *coli* strain BL21-Gold(DE3) was cotransformed with pET_AaLS-R21H-E32H-D6H-I125I and pACYC-Ptet_cpAaLS-tev-GFP-ssrA vectors, and protein production was induced with IPTG (0.2 mM) and tetracycline (0.5 µg/mL).¹⁹

### Size-exclusion chromatography

The proteins isolated using affinity chromatography was placed in a target buffer using an Amicon-15 ultracentrifugal units (50 k MWCO, Merck-Millipore). 20 mM sodium-phosphate buffer (pH7.5) containing 150 mM NaCl, 6 mM KCl, and 1 mM EDTA, referred as "the phosphate buffer"; 50 mM HEPES-NaOH buffer (pH 7.5) containing 150 mM NaCl and 1 mM EDTA, referred to as "the HEPES buffer"; or Tris-HCl buffer (pH 7.5) containing 150 mM NaCl and 1 mM EDTA were tested. The protein concentration was adjusted to 200 µM (as respect to monomer) and kept in a corresponding buffer for >18 hours. 250 µL of 200 µM protein samples were subjected to a Superose 6 increase 10/300 (Cytiva) column using a Biorad NGC pump system and flow rate of 0.75 mL/min.

### Negative-stain transmission electron microscopy (TEM)

The protein solution (0.05 mg/mL, 4 µL) was placed on a glow-discharged carbon coated copper grid (EM resolution). The excess of the solution was removed using filter paper followed by incubation with 1% phosphotungstic acid. Samples were visualized on a JEOL JEM-1230 electron microscope with 80 kV operation. Images were analyzed using software Image J.

### Cryogenic transmission electron microscopy

Protein solution (1.0 mg/mL, ~3 µL) was applied to glow-discharged TEM grids (Quantifoil R2/1, Cu 200 mesh) and plunge-frozen in liquid ethane by a Vitrobot Mark IV (Thermo Fisher Scientific) using the following parameters: humidity, 95%; temperature, 4°C; wait time, 30 s; blot total, 2; blot force, 3; blot time, 3s. Cryo-EM micrographs were collected at the National Cryo-EM Centre SOLARIS (Krakow, Poland) on a Titan Krios cryo-electron microscope equipped with a Falcon 4 detector (Thermo Fisher Scientific), operating at a 300 kV accelerating voltage, a 190,000 × magnification, and a corresponding pixel size of 0.6575 Å. Data were anlyzed using software CryoSparc.

### Dynamic light scattering

Protein solution (0.5 mg/mL) was prepared in the corresponding buffers using ultrafiltration as described above and transferred to a Prometheus high-sensitivity glass capillary sealed with a dedicated sealing paste. For phosphate titration, the buffers containing each phosphate concentration were prepared by mixing the phosphate buffer and the HEPES buffer at appropriate ratio. For experiments using phosphate derivatives and analogs, 50 mM HEPES-NaOH buffer (pH 7.4) containing 150 mM NaCl and 20 mM phosphate derivatives or analogs (pH adjusted to 7.4 by NaOH, if required) was used. Dynamic light scattering (DLS) measurements were performed on a Prometheus PANTA instrument (NanoTemper Technologies) at 25 °C. Datasets were analyzed and merged using the PR.PantaAnalysis software. The measurements were triplicate and means ± standard deviations are provided.

### TEV protease cleavage assay

AaLS cages encapsulating GFP via a peptide containing TEV protease recognition sequence (~2.4 GFP per AaLS cage on average) were prepared in the PBS-E and the HEPES buffers containing 150 mM NaCl. The reactions were initiated upon the addition of TEV protease²⁸⁻³⁰ (10 equiv. to GFP) to the GFP-containing AaLS cage samples and incubated at 25°C for 36 hours. Control samples were mixed with buffer instead of protease. After the incubation, each sample was prepared in SDS-PAGE loading buffer (62.5 mM Tris-HCl (pH 6.8), 2% sodium dodecyl sulfate, 10% glycerol, 5% β-mercaptoethanol, 0.02 % bromophenol blue), followed by heat denaturation. The samples (~1 µg with respect to GFP) were loaded onto 15% polyacrylamide gels and electrophoresed. Protein bands were visualized using ReadyBlue Protein Gel Stain (Sigma-Aldrich).

References:
1. Edwardson, T. G. W.; Levasseur, M. D.; Tetter, S., et al., Chem. Rev. 2022, 122, 9145-9197.
2. Wang, Y.; Douglas, T., Curr. Opin. Colloid Interface Sci. 2021, 51, 101395.
3. Aumiller, W. M.; Uchida, M.; Douglas, T., Chem. Soc. Rev. 2018, 47, 3433-3469.
4. Ritsert, K.; Huber, R.; Turk, D., et al., J. Mol. Biol. 1995, 253, 151-67.
5. Zhang, X.; Meining, W.; Fischer, M., et al., J. Mol. Biol. 2001, 306, 1099-114.
6. Shenton, W.; Mann, S.; Colfen, H., et al., Angew. Chem. Int. Ed. 2001, 40, 442-445.
7. Levasseur, M. D.; Mantri, S.; Hayashi, T., et al., ACS Chem. Biol. 2021, 16, 838-843.
8. Min, J.; Kim, S.; Lee, J., et al., RSC Adv. 2014, 4, 48596-48600.
9. Ra, J. S.; Shin, H. H.; Kang, S., et al., Clin. Exp. Vaccine Res. 2014, 3, 227-34.
10. Kaster, M. A.; Levasseur, M. D.; Edwardson, T. G. W., et al., ACS Appl. Bio Mater. 2023, 6, 591-602.
11. Azuma, Y; Hilvert, D., Enzyme Encapsulation in an Engineered Lumazine Synthase Protein Cage. In Methods Mol. Biol., Udit, A. K., Ed. Springer New York: New York, NY, 2018; pp 39-55.
12. Azuma, Y; Zschoche, R.; Tinzl, M., et al., Angew. Chem. Int. Ed. 2016, 55, 1531-4.
13. Azuma, Y; Bader, D. L. V.; Hilvert, D., J. Am. Chem. Soc. 2018, 140, 860-863.
14. Frey, R.; Mantri, S.; Rocca, M., et al., J. Am. Chem. Soc. 2016,138, 10072-5.
15. Azuma, Y; Edwardson, T. G. W.; Hilvert, D., Chem. Soc. Rev. 2018, 47, 3543-3557.
16. Seebeck, F. P.; Woycechowsky, K. J.; Zhuang, W., et al., J. Am. Chem. Soc. 2006, 128, 4516-7.
17. Wörsdörfer, B.; Woycechowsky, K. J.; Hilvert, D., Science 2011, 331, 589-92.
18. Lilavivat, S.; Sardar, D.; Jana, S., et al., J. Am. Chem. Soc. 2012, 134, 13152-5.
19. Azuma, Y; Herger, M.; Hilvert, D., J. Am. Chem. Soc. 2018, 140, 558-561.
20. Terasaka, N.; Azuma, Y; Hilvert, D., Proc. Natl. Acad. Sci. U. S. A. 2018, 115, 5432.
21. Koziej, L.; Gawin, A.; Azuma, Y, Lumazine Synthase Nanocompartments. In Microbial Production of High-Value Products, Rehm, B. H. A.; Wibowo, D., Eds. Springer International Publishing: Cham, 2022; pp 335-355.
22. Wörsdörfer, B.; Pianowski, Z.; Hilvert, D., J. Am. Chem. Soc. 2012,134, 909-11.
23. Tetter, S.; Terasaka, N.; Steinauer, A., et al., Science 2021, 372, 1220-1224.
24. Azuma, Y; Edwardson, T. G. W.; Terasaka, N., et al., J. Am. Chem. Soc. 2018, 140, 566-569.
25. Azuma, Y; Zschoche, R.; Hilvert, D., J. Biol. Chem. 2017, 292, 10321-10327.
26. Sasaki, E.; Böhringer, D.; van de Waterbeemd, M., et al., Nat. Commun. 2017, 8, 14663.
27. Ladenstein, R.; Fischer, M.; Bacher, A., FEBSJ. 2013, 280, 2537-63.
28. Correnti, C. E.; Gewe, M. M.; Mehlin, C., et al., Nat. Struct. Mol. Biol. 2018, 25, 270-278.
29. Cabrita, L. D.; Gilis, D.; Robertson, A. L., et al., Protein Sci. 2007, 16, 2360-2367.
30. Kapust, R. B.; Tözsér, J.; Fox, J. D., et al., Protein Eng. Des. Sel. 2001, 14, 993-1000.

## Claims

1. A mutant of the lumazine synthase from *Aquifex aeolicus* having amino acid sequence consisting of: 
(SEQ ID NO:3)

MEIYEGKLTA EGLRFGIVAS X1FNHALVDRL VX2GAIHCIVX5 X6GGREEDITL VRVPGSWEIP

VAAGELARKE DIDAVIAIGV LIRGATPHFD YIASEVSKGL ANLSLELRKP ITFGVITADT

X3EQAX4ERAGT KHGNKGWEAA LSAIEMANLF KSLR
 wherein: X₁ is cysteine or a cysteine ortholog, X₂ is a non-negatively charged amino acid, at least one of X₃ or X₄ is a non-hydrophobic amino acid, X₅ is arginine or any another amino acid and X₆ is histidine or any another amino acid.

2. The mutant of claim 1, having amino acid sequence consisting of: 
(SEQ ID NO:2)

MEIYEGKLTA EGLRFGIVAS X1FNHALVDRL VX2GAIHCIVR HGGREEDITL VRVPGSWEIP

VAAGELARKE DIDAVIAIGV LIRGATPHFD YIASEVSKGL ANLSLELRKP ITFGVITADT

X3EQAX4ERAGT KHGNKGWEAA LSAIEMANLF KSLR
 wherein X₁, X₂, X₃ and X₄ have been defined above.

3. The mutant of claim 1, which comprises a amino acid sequence selected among of sequences presented as SEQ. ID. NO. 6-14.

4. The mutant of any one of claims 1 to 3, which further comprises a short peptide tag, wherein said peptide tag is preferably a strep tag consisting of an amino acid sequence of WSHPQFEK.

5. A protein cage comprising the mutant of the lumazine synthase from *Aquifex aeolicus* according to any one of claims 1-4.

6. A inclusion complex comprising the protein cage of claim 5 and a cargo molecule, wherein said cargo molecule is encapsulated in said protein cage.

7. A method for opening of the protein cage of claim 5 or the inclusion complex of claim 6 comprising the step of removal of oxides.

8. The method of claim 7 comprising the step of addition of phosphate antagonists.

9. A method for closing of the protein cage of claim 5 or the inclusion complex of claim 6 comprising the step of addition of oxides.
